# EUROPEAN PATENT APPLICATION

(11) **EP 0 841 066 A1**
(43) Date of publication of application: **13.05.1998**
(21) Application number: 96916347.6
(22) Date of filing: 07.06.1996
(51) Int. Cl.: A61K 38/18, A61K 38/19, A61K 47/18, A61K 47/26, A61K 47/34, A61K 47/42

(54) **STABLE FREEZE-DRIED COMPOSITION CONTAINING TPO**

(30) Priority: 08.06.1995 JP 142075/95
(71) Applicant: Kirin Brewery Company, Ltd., Tokyo 104 (JP)
(72) Inventor: NOMURA, Hideaki, Kirin Brewery Company, Limited, Gunma-ken 370-12 (JP)
(74) Representative: UEXKÜLL & STOLBERG
(86) International application number: JP9601561
(87) International publication number: WO9641642

(57) **Abstract**

The present invention relates to a thrombopoietin (TPO)-containing lyophilized composition which comprises a TPO protein and a saccharide as a pharmaceutically acceptable additive. By the invention, decrease in the activity of TPO as an active ingredient can be prevented or inhibited during long-term preservation.

## Description

### BACKGROUND OF THE INVENTION

### 1. Field of the Invention

This invention relates to a composition which contains a TPO protein, more particularly to a stable TPO-containing lyophilized composition.

### 2. Disclosure of Related Art

Human TPO (thrombopoietin) is a protein cloned as an Mpl ligand which is a member of the cytokine receptor superfamily (de Sauvage *et al*., *Nature* (London), vol.369, pp.533 - 565 (1994); Bartley, T.D. *et al*., *Cell*, vol.77, pp.1117 - 1124 (1994)). The Mpl ligand can be detected in sera and blood plasmas of animals (including human, mouse and canine) suffering from thrombocytopenia, and its relation to the production of megakaryocytes and platelets has already been confirmed.

With the aim of developing a therapeutic agent for thrombocytopenia, the present inventors have purified rat TPO from plasmas of thrombocytopenic rats by using as an indication, an activity that stimulates the production of megakaryocytes from megakaryocyte progenitor cells highly purified from rat bone marrow, and have succeeded in cloning of rat TPO cDNA and human TPO cDNA based on a partial amino acid sequence of the rat TPO thereby obtaining homogeneous human TPO in a large quantity by recombinant DNA techniques (H. Miyazaki *et al*., *Exp*. *Hematol*., vol.22, p.838 (1994)). The thus successfully obtained human TPO has the same amino acid sequence as that of the aforementioned factor obtained as a human Mpl ligand (SEQ ID NO: 1 in SEQUENCE LISTING described below).

The present inventors have found that the TPO of the present invention was effective in treatment of thrombocytopenia, because the inhibition of decrease in platelets, thrombocytopoiesis enhancement of increase in platelets, and enhancement of hematopoietic function were observed when said human TPO was administered to mice with thrombocytopenia in which bone marrow suppression has been induced by administration of an anticancer agent or immunosuppressant or by radiation or BMT.

TPO is used in an extremely small amount due to its high activity. Namely, it is normally administered several times a day in a dose of from 0.05 µg/kg body weight to 1 mg/kg body weight, preferably from 0.5 µg/kg body weight to 50 µg/kg body weight, as the active ingredient depending on conditions, sexes and administration routes. Thus, it is required to produce pharmaceutical preparations having an extremely small quantity of TPO, so the provision of the stable pharmaceutical preparations is demanded that can fully prevent decrease in the activity of the active ingredient.

The present inventors have studied on the development of a stable TPO protein composition which is preservable for a long period of time. As a result, it has now been found that the addition of a pharmaceutically acceptable saccharide to a TPO protein followed by lyophilization led to a considerably improved stability of the TPO protein, that the addition of a surfactant besides the saccharide was effective for further improvement of the stability of the TPO-containing lyophilized composition and also for improvement of the solubility of the TPO-containing lyophilized composition when reconstituted, and that the further addition of an amino acid or a protein could improve the stability of the TPO-containing lyophilized composition more efficiently.

### SUMMARY OF THE INVENTION

According to the present invention, there is provided a TPO-containing lyophilized composition which comprises a TPO protein and a pharmaceutically acceptable saccharide, and optionally at least one pharmaceutically acceptable additive selected from the group consisting of a surfactant, an amino acid and a protein.

### BRIEF DESCRIPTION OF THE DRAWINGS

Fig. 1 is a graph showing stabilization of a TPO protein when a saccharide (mannitol, lactose, sucrose or maltose) is added, wherein the % residual TPO is used as an indication of the stabilization of TPO.

Fig. 2 is a graph showing stabilization of the TPO protein when a surfactant (polysorbate 20 or polysorbate 80) is added in addition to the saccharide, wherein the % residual TPO is used as an indication of the stabilization of TPO.

Fig. 3 is a graph showing stabilization of the TPO protein when an amino acid (arginine or glycine) or a protein (gelatin) is added in addition to the saccharide and surfactant, wherein the % residual TPO is used as an indication of the stabilization of TPO.

### DETAILED DESCRIPTION OF THE INVENTION

As the TPO used in the present invention, a protein having the amino acid sequence shown in SEQ ID NO: 1 can be used. Methods for preparing the TPO are not particularly limited, but the TPO product is a protein isolated in a high purity. Also used as the TPO of the present invention is a protein having an amino acid sequence partially modified (by substitution, deletion, insertion and/or addition) in the amino acid sequence shown in SEQ ID NO: 1, provided that it maintains the TPO activity.

In other words, a protein whose amino acid sequence is substantially the same amino acid sequence shown in SEQ ID NO: 1 can also be used. The term "substantially the same amino acid sequence shown in SEQ ID NO: 1" as used herein means that the "amino acid sequence resulting from partial substitution, deletion, insertion and/or addition of the amino acid sequence shown in SEQ ID NO: 1, provided that it maintains the TPO activity", is included in addition to the amino acid sequence shown in SEQ ID NO: 1.

The present inventors have confirmed that human TPO can keep its activity even if amino acid residues of the C-terminal side of the amino acid sequence shown in SEQ ID NO: 1 are deleted up to the position 152 residue, or even if those of its N-terminal side are deleted up to the position 6. Illustrative data are shown in Table 1.

**Table 1**

| Derivative | Activity of TPO |
|---|---|
| positions 1 - 231 | + |
| positions 1 - 211 | + |
| positions 1 - 191 | + |
| positions 1 - 171 | + |
| positions 1 - 163 | + |
| positions 1 - 157 | + |
| positions 1 - 156 | + |
| positions 1 - 155 | + |
| positions 1 - 154 | + |
| positions 1 - 153 | + |
| positions 1 - 151 | + |
| positions 1 - 150 | - |
| positions 7 - 163 | + |
| positions 8 - 163 | - |
| positions 13 - 231 | - |

Thus, the TPO used in the present invention also includes a protein which contains an amino acid sequence corresponding to the positions 7 to 151 of the amino acid sequence shown in SEQ ID NO: 1 and has the TPO activity. More particularly, proteins which respectively comprise positions 1 to 231, positions 1 to 211, positions 1 to 191, positions 1 to 171, positions 1 to 163, positions 1 to 157, positions 1 to 156, positions 1 to 155, positions 1 to 154, positions 1 to 153, positions 1 to 151 and positions 7 to 163 of the amino acid sequence shown in SEQ ID NO: 1 can be exemplified as the TPO of the present invention.

Also included in the TPO of the present invention is a protein which comprises an amino acid sequence having a substitution, deletion, insertion and/or addition of at least one amino acid residue inside or outside of the aforementioned positions 7 to 151 sequence, to the extent that the TPO activity is not spoiled.

Other examples of the TPO used in the present invention include a protein in which at least the 1-position serine residue and the 3-position alanine residue of human TPO having the amino acid sequence of SEQ ID NO: 1 are respectively substituted by an alanine residue and a valine residue, a protein in which the 25-position arginine residue is substituted by an asparagine residue, a protein in which the 33-position histidine residue is substituted by a threonine residue, a protein in which the 25-position arginine residue is substituted by an asparagine residue and the 231-position glutamic acid residue is substituted by a lysine residue, and proteins in which a polypeptide:
ThrSerIleGlyTyrProTyrAspValProAspTyrAlaGlyValHisHisHisHisHisHis
is added to each C-terminus of the above described proteins. Further included are proteins having the deletion and/or addition of at least the following amino acid residues in the sequence shown in SEQ ID NO:1, namely, a protein in which the 33-position histidine residue is deleted, a protein in which the 116-position glycine residue is deleted, a protein in which the 117-position arginine residue is deleted, a protein in which a threonine residue is inserted between the 33-position histidine residue and the 34-position proline residue, a protein in which an alanine residue is inserted between the 33-position histidine residue and the 34-position proline residue, a protein in which a glycine residue is inserted between the 33-position histidine residue and the 34-position proline residue, a protein in which a glycine residue is inserted between the 33-position histidine residue and the 34-position proline residue and the 38-position proline residue is substituted by a serine residue, a protein in which an asparagine residue is inserted between the 116-position glycine residue and the 117-position arginine residue, a protein in which an alanine residue is inserted between the 116-position glycine residue and the 117-position arginine residue, and a protein in which a glycine residue is inserted between the 116-position glycine residue and the 117-position arginine residue.

Still further examples of the TPO proteins of the present invention are: a protein in which at least the 129-position leucine residue is substituted by an arginine residue, a protein in which the 133-position histidine residue is substituted by an arginine residue, a protein in which the 143-position methionine residue is substituted by an arginine residue, a protein in which the 82-position glycine residue is substituted by a leucine residue, a protein in which the 146-position glycine residue is substituted by a leucine residue, a protein in which the 148-position serine residue is substituted by a proline residue, a protein in which the 59-position lysine residue is substituted by an arginine residue, and a protein in which the 115-position glutamine residue is substituted by an arginine residue.

Also included as the TPO proteins of the present invention are proteins in which methionine and lysine residues are respectively added to the positions -2 and -1 of the human TPO protein having the amino acid sequence shown in SEQ ID NO: 1 or of the above described derivatives; and proteins in which a methionine residue is attached at the protein -1 of the human TPO protein having the amino acid sequence shown in SEQ ID NO: 1 or of the above described derivatives.

Preferably, the TPO proteins used in the present invention may be obtained by isolating and purifying them from host cells transformed with a recombinant vector containing their cDNA, chromosomal DNA or chemically synthesized DNA. As the host, procaryotic cells (e.g., bacteria, preferably *Escherichia coli*) or eucaryotic cells (e.g., yeasts, insects or mammals) can be used. Examples of the mammalian cells include COS cells, Chinese hamster ovary (CHO) cells, X63.6.5.3. cells, C-127 cells, BHK (Baby Hamster Kidney) cells, human cells (e.g., HeLa cells), and so on. Examples of the yeast include a baker's yeast (*Saccharomyces cerevisiae*), a methanol assimilating yeast (*Pichia pastoris*), and the like. Examples of the insect cells include silkworm culture cells (e.g., Sf21 cells), and the like.

Examples of the production of the TPO of the present invention by use of CHO cells and by use of *E. coli* are described in Reference Examples 1 and 2, respectively.

When the TPO protein is produced using *E. coli*, it can be obtained by a method in which a DNA fragment coding for the protein, provided with a restriction site(s) and/or added to DNA capable of facilitating its expression, is inserted into an appropriate expression vector, procaryotic cells (such as bacterial cells, preferably *E. coli*) transformed with the vector are cultured, and then the thus produced protein having TPO activity is isolated and purified. When *E. coli* is used as the host, codons suitable for the expression in *E. coli* (i.e., preferential codons) may be integrated.

Examples of the vector to be used in the transformation of *E. coli* include pKC30 (Shimatake H. and M. Rosenberg, *Nature*, 292, pp.128 - 132, 1981), pTrc99A (Amann E. *et al*., *Gene*, 108, pp.193 - 200, 1991), pCFM536 (ATCC No. 39934; see JP-A-60-501988), and the like.

For example, when a TPO protein having the 1 - 332 amino acid sequence shown in SEQ ID NO: 1 is produced, a DNA fragment coding for the 1 - 332 amino acid sequence is synthesized; a DNA sequence which encodes a methionine residue and a lysine residue is added to its N-terminus and a DNA sequence that becomes a *Xba*I site is further added to a upstream site of said DNA sequence; and a DNA sequence which encodes a stop codon is added to its C-terminus and a DNA sequence that becomes a *Hin*dIII site is further added to a downstream site of said DNA sequence.

By treating this DNA fragment with *Xba*I/*Hin*dIII, a DNA fragment shown in SEQ ID NO: 2 for example can be obtained. The thus obtained DNA fragment is cloned into pCFM536 (ATCC No. 39934; see JP-A-60-501988) digested in advance with *Xba*I and *Hin*dIII, and *E. coli* JM109 pretransformed with pMW1 (ATCC No. 39933) is made into a transformant containing the expression vector for expression of a TPO protein.

Expression of the expression plasmid pCFM536 may be controlled by λPL promoter under regulation of a cI857 repressor gene. The transformant obtained in this manner is cultured to isolate and purify an expressed TPO protein.

By carrying out a cathepsin treatment or the like during the purification process, the methionine-lysine residues added to the N-terminus are cleaved out to obtain the TPO protein having the 1 - 332 amino acid sequence.

In this connection, a plasmid pHTF1 having a DNA fragment coding for the 1 - 332 amino acid sequence (see SEQ ID NO: 3), transformed into *E. coli* strain DH5, has been deposited under the terms of the Budapest Treaty on March 24, 1994, with the National Institute of Bioscience and Human Technology, Agency of Industrial Science and Technology, Ministry of International Trade and Industry, Japan, under the Accession No. FERM BP-4617. The same plasmid pHTF1 has also been deposited with the Chinese depositary authority CCTCC (Lou Jia Shan, Wuhan 430072, China) under Accession No. CCTCC-M95004.

According to the present invention, saccharides, surfactants, amino acids and proteins can be exemplified as the additives useful in preparing the stable TPO-containing lyophilized composition. Examples of these additives improving stability of the TPO composition include, but not limited to, the following materials:

As the saccharides, mannitol, lactose, sucrose, maltose, glucose, inositol, xylose, sorbitol, fructose, galactose, ribose, mannose, cellobiose, cyclodextrin and the like can be used.

As the surfactants, polyoxyethylene hydrogenated castor oil, polyoxyethylene castor oil, polyoxyethylene sorbitan fatty acid esters such as polysorbate 80, polyoxyethylene sorbitan monolaurate (alias: polysorbate 20) and the like, polyoxyethylene polyoxypropylene glycol, sorbitan fatty acid esters such as sorbitan monooleate and the like, sucrose fatty acid esters such as sucrose monolauric acid ester and the like, aromatic quaternary ammonium salts such as benzethonium chloride, benzalkonium chloride and the like, sodium caprylate, sodium sulfite and the like can be used.

As the amino acids, glycine, alanine, methionine, cysteine, asparagine, aspartic acid and a salt thereof, glutamine, glutamic acid and a salt thereof, histidine, lysine, arginine and the like can be used.

As the proteins, gelatin, human serum albumin, casein, collagen, human serum globulin and the like can be used.

The additives used in the present invention can be used within the range from 10 to 10000 parts by weight in the case of a saccharide, from 0.01 to 10 parts by weight in the case of a surfactant, from 1 to 1000 parts by weight in the case of an amino acid, or from 1 to 1000 parts by weight in the case of a protein, relative to one part by weight of the TPO contained in the TPO-containing lyophilized composition.

In addition, the TPO-containing lyophilized composition of the present invention may also contain a diluent, a solubilizing agent, an antiseptic agent, an antioxidant, an excipient, a isotonicity agent and the like depending on its preparation purposes.

The lyophilizing technique used in the present invention can be effected in the usual way. For example, the TPO composition of the present invention in the form of an aqueous solution is dispensed in 1 ml portions into vials and then friezed in the drying chamber of a freeze dryer cooled in advance to -40°C. When sufficient friezing of the contents in vials is confirmed (generally 2 to 3 hours), the vacuum pump is run to remove moisture from the friezed bodies in the vials by sublimation. In this case, the drying is carried out at a low temperature by setting a temperature in the drying chamber to -10°C. When the drying step is completed (generally 0.5 day to 2 days), finish drying is carried out (generally several hours to 1 day) in the drying chamber at room temperature to obtain the TPO lyophilized composition of interest.

### EXAMPLES

The present invention will be illustrated by Examples, Test Example and Referrence Examples set forth below.

### Example 1

250 µg of the TPO which can be obtained by the method described in Reference Example 1 and 30 mg of mannitol were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Example 2

250 µg of the TPO which can be obtained by the method described in Reference Example 1 and 30 mg of lactose were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Example 3

250 µg of the TPO which can be obtained by the method described in Reference Example 1 and 30 mg of sucrose were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Example 4

250 µg of the TPO which can be obtained by the method described in Reference Example 1 and 30 mg of maltose were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Example 5

250 µg of the TPO which can be obtained by the method described in Reference Example 1, 30 mg of maltose and 40 µg of polysorbate 20 were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Example 6

250 µg of the TPO which can be obtained by the method described in Reference Example 1, 30 mg of maltose and 40 µg of polysorbate 80 were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Example 7

250 µg of the TPO which can be obtained by the method described in Reference Example 1, 29 mg of maltose, 40 µg of polysorbate 80 and 1 mg of arginine were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Example 8

250 µg of the TPO which can be obtained by the method described in Reference Example 1, 29 mg of maltose, 40 µg of polysorbate 80 and 1 mg of glycine were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Example 9

250 µg of the TPO which can be obtained by the method described in Reference Example 1, 29 mg of maltose, 40 µg of polysorbate 80 and 1 mg of gelatin were dissolved in 1 ml of 5 mM phosphate buffer at pH 6.0, the thus prepared aqueous solution was dispensed in 1 ml portions into vials and lyophilized, and then the vials were sealed to obtain a lyophilized composition.

### Comparative Example 1

250 µg of the TPO which can be obtained by the method described in Reference Example 1 was dissolved in 1 ml of 5 mM phosphate buffer at pH 6.0, the thus prepared aqueous solution was dispensed in 1 ml portions into vials, and then the vials were sealed to obtain an aqueous solution composition.

The kinds and contents of the additives used in the TPO-containing compositions prepared in Examples 1 to 9 are summarized in Table 2.

**Table 2**

| Example No. | Saccharide | Surfactant | Amino acid | Protein |
|---|---|---|---|---|
| 1 | mannitol 3% | ― | ― | ― |
| 2 | lactose 3% | ― | ― | ― |
| 3 | sucrose 3% | ― | ― | ― |
| 4 | maltose 3% | ― | ― | ― |
| 5 | maltose 3% | polysorbate 20 0.004% | ― | ― |
| 6 | maltose 3% | polysorbate 80 0.004% | ― | ― |
| 7 | maltose 2.9% | polysorbate 80 0.004% | arginine 0.1% | ― |
| 8 | maltose 2.9% | polysorbate 80 0.004% | glycine 0.1% | ― |
| 9 | maltose 2.9% | polysorbate 80 0.004% | ― | gelatin 0.1% |

Test Example is described in the following.

In the Test Example, the % residual TPO was determined by the reverse phase liquid chromatography method and the biological assay in the following manner.

### Reverse phase liquid chromatography method

Each sample containing 1 µg or more of TPO is injected into a C8 reverse phase column (4.6 mm × 250 mm) using n-propanol and trifluoroacetic acid as the mobile phase, and the residual amount of TPO is measured under the following gradient conditions (see Table 3).

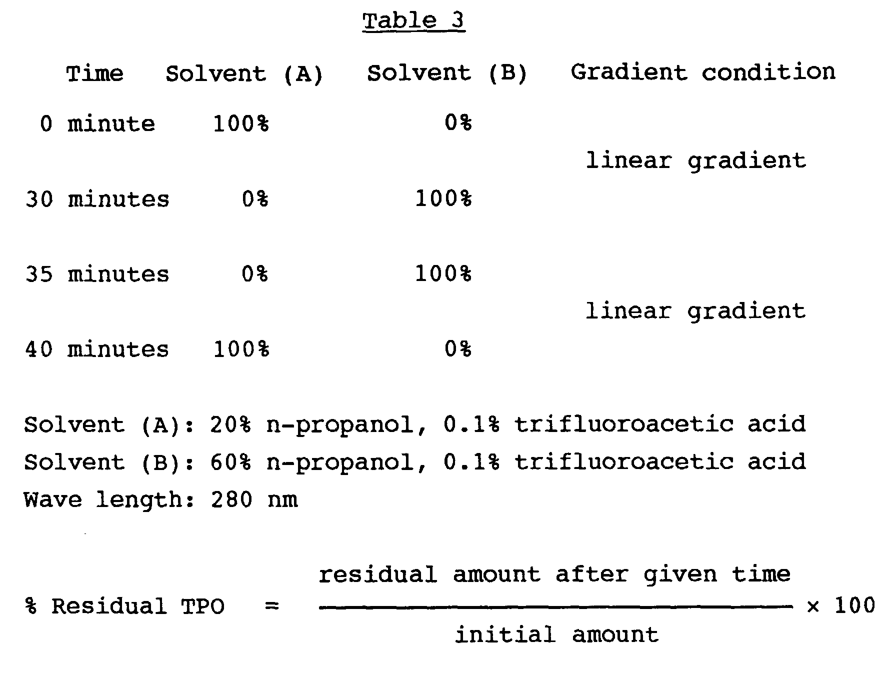

### Biological assay method

### (a) Assay system using 32D-hu-mpl⁺ cells (32D-mpl assay):

### [Assay method]

### (1) Establishment of mouse 32D-hu-mpl⁺ cells for use in the assay

A full length human Mpl receptor gene (Vigon, I. *et al*., *PNAS*, vol.89, pp.5640 - 5644 (1992)) is subcloned into an expression vector containing a transcriptional promoter derived from Moloney Murine Sarcoma virus LTR.

6 µg of this recombinant vector and 6 µg of an amphotrophic retroviral packaging construct (Landau, N.R. and Littman D.R., *J. Virology*, vol.66, pp.5110 - 5113 (1992)) are transfected into 3 × 10⁶ of 293 cells using CaPO₄ Mammalian Transfection Kit (manufactured by Stratagene). The cells were retransfected after 2 days and again after 4 days. On the day after the final transformation, the 293 cells are cocultivated with the IL-3-dependent murine cell line (32D, clone 23; Greenberger *et al*., *PNAS*, vol.80, pp.2931 - 2936 (1983)). After 24 hours of the culture, the 32D cells are rescued and isolated by means of the density-gradient using a BSA gradient (Path-o-cyte; Mills Inc.). Cells are expanded in the presence of 1 ng/ml murine IL-3 and then are selected using 20% APK9 (Vignon *et al*., *PNAS*, vol.89, pp.5640 - 5644 (1992); Landau, N.R. and Littman D.R., *J*. *Virology*, vol.66, pp.5110 - 5113 (1992)). Cells having human Mpl receptor expressed on the cell surface (32D-hu-mpl⁺ cells) are sorted by FACS using a rabbit anti-serum to the human Mpl receptor peptide.

### (2) Assay using 32D-hu-mpl⁺ cells

The 32D-hu-mpl⁺ cells subcultured in the presence of mouse IL-3 are recovered, washed well to remove the mouse IL-3, and then again suspended in a growth medium (MEM medium containing 10% FCS).

On the other hand, a standard and a sample to be tested are separately diluted with the growth medium, and the dilutions are dispensed in 100 µl portions into wells of a plate. The 32D-hu-mpl⁺ cell suspension prepared as above is diluted with the growth medium to a concentration of 1 × 10⁵ cells/ml and dispensed in 100 µl portions into wells of the aforementioned plate. This plate is incubated for 48 hours in a highly humidified incubator at 37°C in 10% CO₂. Thereafter, MTS solution is added in an amount of 20 µl to each well of the plate which is subsequently incubated for 4 hours in a highly humidified incubator at 37°C in 10% CO₂. After the incubation, an absorbance is measured at 490 nm using a microplate reader.

### (b) Assay system using a human megakaryoblast cell line (M-07e assay):

It is known that cells of a human megakaryoblast cell line, M-07e, grow in response to GM-CSF, IL-3, SCF, IL-2 or the like (Avanzi *et al*., *J. Cell*. *Physiol*., vol.145, pp.458 - 464, (1990); Kiss *et al*., *Leukemia*, vol.7), and it was revealed that these cells also respond to TPO.

### [Assay method]

M-07e cells subcultured in the presence of GM-CSF are recovered, rinsed well and then resuspended in IMDM culture medium containing 10% FCS. The resulting M-07e cell suspension is dispensed in an amount of 10⁴ cells/well into a 96 well tissue culture plate, and each well is further supplied with a standard or a sample to be assayed, thereby adjusting the final volume to 200 µl/well. The plate is put in a 5% CO₂ incubator and then incubated for 3 days at 37°C. Four hours before completion of the culture on Day 3, 1 µCi (37 KBq) of ³H-thymidine is added to each well and, after completion of the culture, the cells are collected on a glass fiber filter using a cell harvester to measure ³H radioactivity with a liquid scintillation counter (for example, Beta Plate manufactured by Pharmacia).

### Test Example

The TPO-containing compositions prepared in Examples 1 to 9 and Comparative Example 1 were stored at 50°C for 1 month. The residual titer of TPO was measured by the reverse phase liquid chromatography method and the biological assay method (a), i.e. the 32D-mpl assay, and the reconstitution of each composition after the storage was observed visually. The results are summarized in Table 4.

**Table 4**

| | % Residual TPO after 1 month of storage at 50°C | |
|---|---|---|
| | Reverse phase liquid chromatography | Biological assay method |
| Example 1 | 76.8 | 45.0 |
| Example 2 | 99.1 | 89.3 |
| Example 3 | 96.6 | 84.0 |
| Example 4 | 97.7 | 69.7 |
| Example 5 | 99.0 | 94.4 |
| Example 6 | 95.6 | 85.1 |
| Example 7 | 97.8 | 96.2 |
| Example 8 | 102.3 | 111.2 |
| Example 9 | 97.0 | 105.3 |
| Comparative Ex. 1 | 59.1 | 39.1 |

As seen in the above table, the stability of TPO is considerably improved when the lyophilization treatment is carried out through addition of a saccharide to TPO. Also, the stability is further improved when the lyophilization treatment is carried out through further addition of at least one pharmaceutically acceptable additive agent selected from the group consisting of surfactants, amino acids and proteins, in addition to the TPO and saccharide. In addition, the TPO-containing lyophilized compositions prepared by adding a surfactant in Examples 5 to 9 showed further improved solubility when reconstituted.

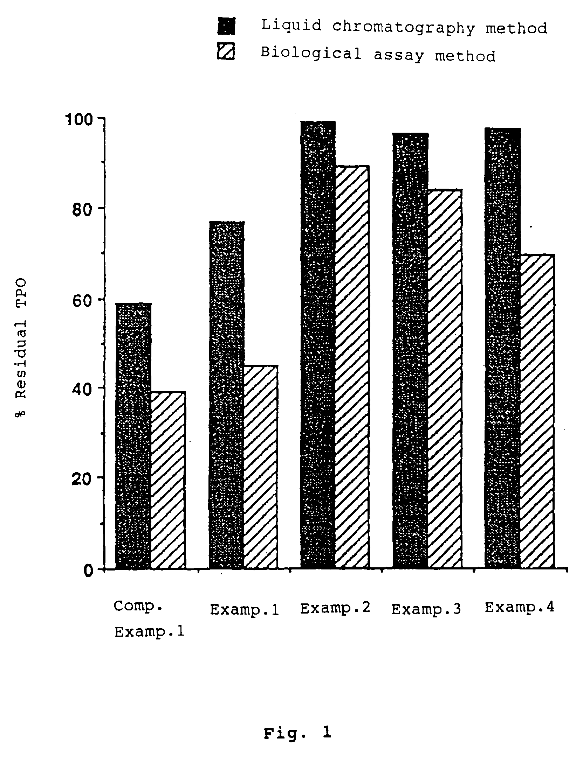

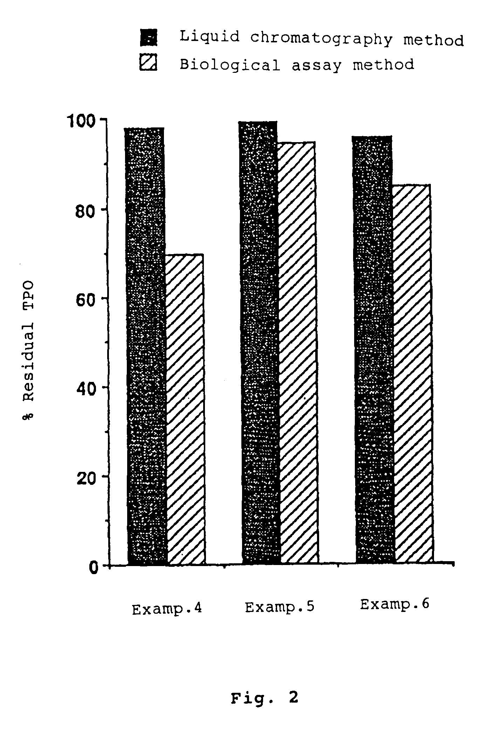

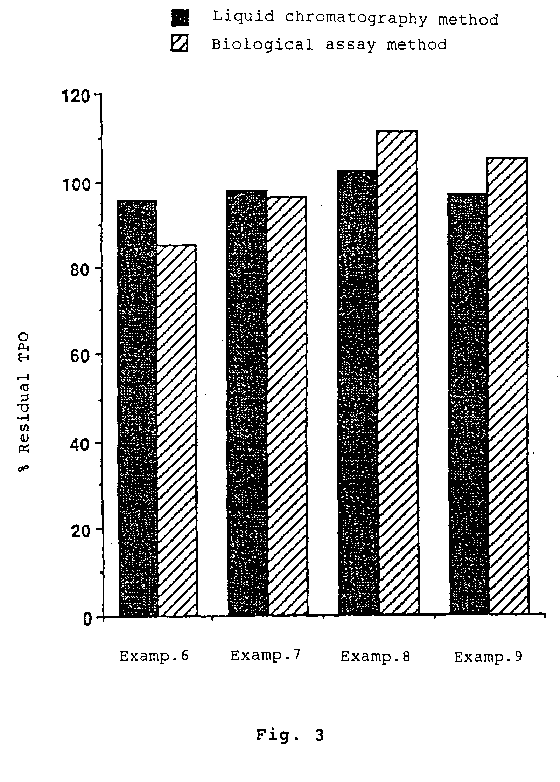

Stabilization effect of saccharides is shown in Fig. 1, stabilization effect of surfactants in Fig. 2, and stabilization effect of amino acids and a protein in Fig. 3, respectively. It has now been found that the stability was improved in all cases in comparison with the additive-free TPO liquid preparation.

The same results as in the biological assay method (a), 32D-mpl assay, were obtained in the method (b), M-07e assay.

The following Reference Examples are provided to illustrate production examples of TPO which is an active ingredient of the present invention.

### Reference Example 1

### Example of the production of TPO(1-332)

(1) CHO cells (dhfr⁻ strain; Urlaub and Chasin, Proc. Natl. Acad. Sci. USA, vol.77, p.4216, 1980) were cultured in plates (6 cm in diameter) (manufactured by Falcon) using an (α-minimum essential medium (α-MEM(-); supplemented with thymidine and hypoxanthine) containing 10% fetal calf serum, and the thus grown cells were then transformed with a plasmid pDEF202-hTPO-P1 by the calcium phosphate method (Cellphect, manufactured by Pharmacia).
   That is, 10 µg of the plasmid pDEF202-hTPO-P1 containing a cDNA insert shown in SEQ ID NO:4 was mixed with 120 µl of buffer A and 120 µl of H₂O and left for 10 minutes at the room temperature. Next, 120 µl of buffer B was added to the resulting solution, mixed again and then left for 30 minutes at room temperature. This DNA solution was added dropwise to the aforementioned plates, followed by 6 hours of cultivation in a CO₂ incubator. After removing the medium from the plates, the culture was washed twice with α-MEM(-) followed by addition of 10% dimethylsulfoxide-containing α-MEM(-), and treated at the room temperature for 2 minutes. Next, a 10% dialyzed fetal calf serum-containing non-selection medium (α-MEM(-), supplemented with hypoxanthine and thymidine) was added to the culture which was then cultured for 2 days, after which the selection was effected using a 10% dialyzed fetal calf serum-containing selection medium (α-MEM(-), without hypoxanthine and thymidine). The selection was carried out by a procedure in which the cells from each of the 6-cm plates were treated with trypsin, divided into five new 10-cm plates or twenty 24-well plates, and then continuously cultured while exchanging the medium with the selection medium every 2 days. The culture supernatant in plates or wells in which cells were grown was assayed for human TPO activity. Cells that the human TPO activity was confirmed in the culture supernatant were divided 1:15 in new plates or wells containing a 25 nM methotrexate-containing selection medium, and the cultivation was continued to effect cloning by allowing methotrexate-resistant cells to grow. In this connection, the transformation of the CHO cells can also be carried out by co-transfection of CHO cells with pHTP-1 and pMG1.
   A CHO cell strain (CHO-DUKXB11) transformed with the plasmid pDEF202-hTPO-P1 has been deposited under the terms of the Budapest Treaty on January 31, 1995 with the National Institute of Bioscience and Human Technology, Agency of Industrial Science and Technology, Ministry of International Trade and Industry, Japan, under Accession No. FERM BP-4988. The above CHO cell line has also been deposited with the Chinese depositary authority CCTCC (Luo Jia Shan, Wuhan 430072, China) under Accession No. CCTCC-C95004.
(2) Culture of human TPO-producing CHO cell line and purification of human TPO:
   A human TPO-producing CHO cell strain (CHO28/1/1/3-C6 strain, resistant to 400 nM MTX) obtained by repeating MTX resistance was cultured in the following manner. The cells were cultured using a DMEM/F-12 medium (GIBCO) containing 400 nM MTX and 10% FCS. The grown cells were peeled off using a trypsin solution, and 1 x 10⁷ of the cells were inoculated into 200 ml of the same medium in a Falcon roller bottle (Falcon 300) and cultured at 37°C at a rotation speed of 1 rpm over 3 days. Thereafter, the culture supernatant was removed by suction, and the remaining cells were rinsed with 50 ml of PBS, suspended in 300 ml of the DMEM/F-12 medium (GIBCO) which has been supplemented with 2 µg/ml of insulin and 10 µM copper sulfate instead of 400 nM MTX and 10% FCS, and then subjected to 4 days of cultivation at 37°C at a rotating speed of 1 rpm to recover a culture supernatant (designated as Harvest-1). Subsequently, 300 ml of the above described production medium was added to the remaining cells which were then cultured at 37°C at a rotation speed of 1 rpm over 4 days to recover a culture supernatant (designated as Harvest-2).

About 220 L of the combined serum-free CHO cell culture supernatants of Harvests-1 and -2 were passed through a 0.5 µm filter (FILTER CARTRIDGE, manufactured by Nihon Pall, Japan) and concentrated to a volume of about 5 L by ultrafiltration (CENTRASETTETM OMEGA 30k cut, manufactured by FILTRON) while simultaneously exchanging the solvent with 10 mM potassium phosphate buffer (pH 6.8). To the concentrate was added 10 µM (final concentration) of a protease inhibitor E-64 (manufactured by Peptide Institute, Japan), and the resulting solution was applied at a flow rate of 100 ml/min to an SP Sepharose FF column (11 cm in diameter and 10 cm in bed height, manufactured by Pharmacia) which has been equilibrated with 10 mM potassium phosphate buffer (pH 6.8). After washing with the equilibration buffer, elution was effected with 1,700 ml of 10 mM potassium phosphate buffer (pH 6.8) containing 0.3 M sodium chloride. The eluate was mixed with 363 g of ammonium sulfate and centrifuged at 12,000 x g for 20 minutes, and the resulting supernatant was loaded at a flow rate of 100 ml/min to a MacroPrep Methyl HIC column (6 cm in diameter and 30 cm in bed height, manufactured by Bio-Rad) which has been equilibrated with 10 mM potassium phosphate buffer (pH 6.8) containing 1.2 M ammonium sulfate. After loading, the column was washed with the equilibration buffer, and elution was effected with 700 ml of 10 mM potassium phosphate buffer (pH 6.8) containing 0.5 M ammonium sulfate. The eluate was mixed with 80 ml of propanol and loaded to a SOURCE 15 RPC column (3.5 cm in diameter and 10 cm in bed height, manufactured by Pharmacia) at a flow rate of 16 ml/min. After loading, the column was washed with 10 mM Tris buffer (pH 7.5) containing 10% propanol (designated as Development solvent A), and elution was effected with a 60-min linear gradient from Development solvent A to 10 mM Tris buffer (pH 7.5) containing 80% propanol ( designated as Development solvent B) until the propanol concentration reached 70%. 192 ml of fractions eluted at around 25 minutes after the beginning of the linear gradient were collected and divided into 2 pools, and each pool was applied to a Superdex 200 pg column (10 cm in diameter and 56 cm in bed height, manufactured by Pharmacia) which has been equilibrated with PBS, and then elution was effected at a flow rate of 40 ml/min. When the eluates were analyzed by SDS-PAGE, a protein having a molecular weight of about 65,000 to about 100,000 which was expected to be TPO was found as a single band at a retention time of around 42 to 52 minutes. A western analysis showed that this protein is TPO. When a portion of this sample was subjected to N-terminal amino acid analysis as well as to amino acid composition analysis, the results revealed that about 230 mg of TPO having the 1-322 amino acid sequence shown in SEQ ID NO:1 was obtained in a highly purified form.

### Reference Example 2

### Example of the production of TPO(1-163)/E. coli in Escherichia coli

### (1) Construction of E. coli expression plasmid pAMG11-hMKT(1-163) for hMKT(1-163) and its expression in E. coli:

To express a protein having an amino acid sequence of the positions 1 through 163 shown in SEQ ID NO:1 (referred to as "TPO(1-163)/E. coli" hereinafter) in *E. coli*, a DNA fragment coding for the amino acid sequence was chemically synthesized using preferential codons for *E. coli*. In addition, a nucleotide sequence which encodes methionine and lysine residues newly added at the N-terminal side was ligated with the DNA fragment, and a DNA sequence encoding a stop codon was added to a site corresponding to the C-terminal side. SEQ ID NO:5 shows an amino acid sequence of the protein encoded by this DNA, namely the protein in which the Met-Lys are attached to the N-terminus of the 1-163 amino acid sequence shown in SEQ ID NO:1 (referred to as "hMKT(1-163)" hereinafter).

The hMKT(1-163) gene fragment synthesized as above has XbaI and HindIII restriction sites at its 5'-end and 3'-end, respectively, and it contains a ribosome binding site, an ATG initiation codon, a sequence encoding the amino acid sequence of hMKT(1-163), and a stop codon.

The above fragment was cloned into the XbaI-HindIII sites of the lactose-inducible expression vector, pAMG11. The pAMG11 vector is a low copy-number plasmid having a pR100-derived replication origin. The expression vector pAMG11 can be obtained from a plasmid pCFM1656 (ATCC No.69576, deposited on February 24, 1994) by causing a series of site-directed base mutations via mutagenesis accompanied with PCR. This plasmid has a BglII site (plasmid bp # 180) starting with immediately at the 5'-side of a plasmid replication promoter, PcopB, followed by a plasmid replication gene. The mutation of base pairs is shown in Table 5.

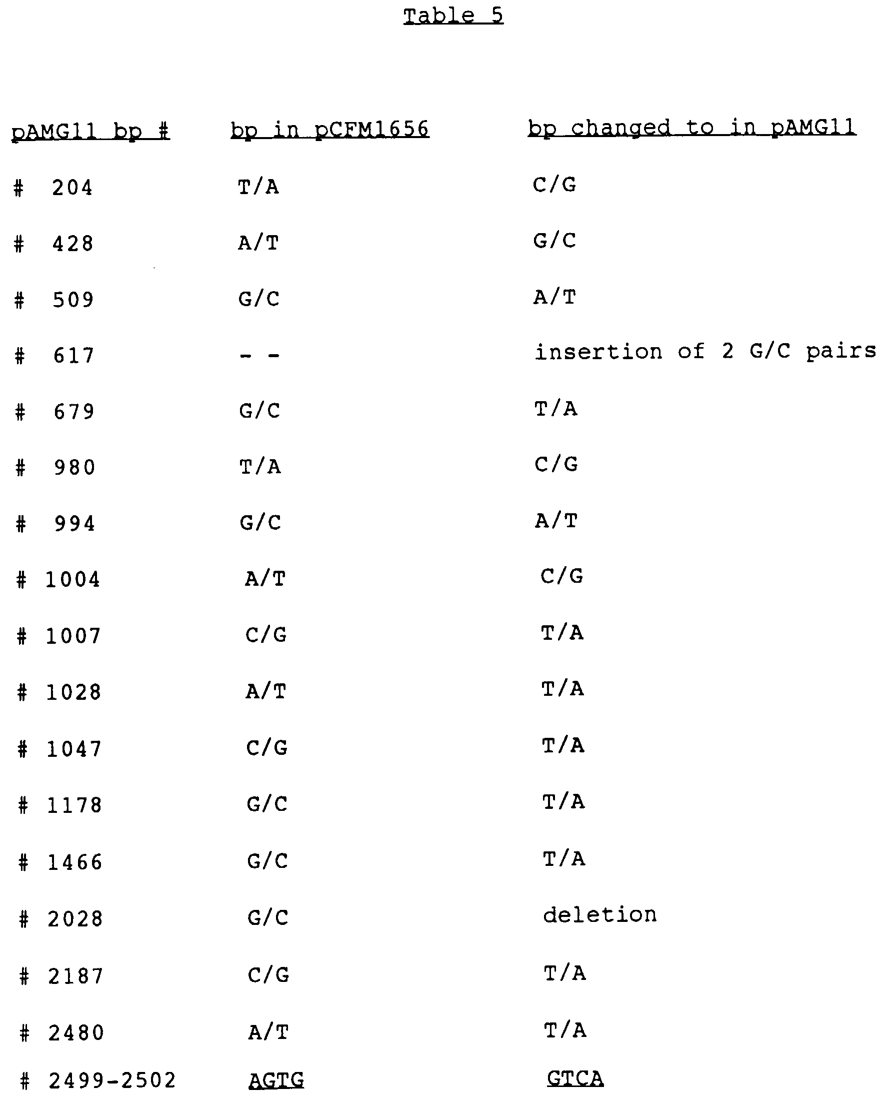

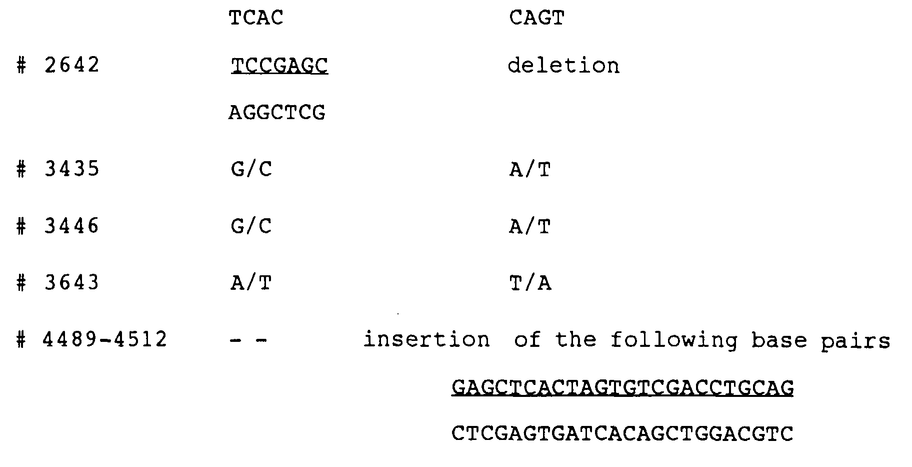

Next, the DNA sequence between the unique AatII and ClaI sites was replaced by the following oligonucleotide.

AatII (#4358)

5'     CTCATAATTTTTAAAAAATTCATTTGACAAATGCTAAAATTCTT-

3' TGCAGAGTATTAAAAATTTTTTAAGTAAACTGTTTACGATTTTAAGAA-

-GATTAATATTCTCAATTGTGAGCGCTCACAATTTAT   3'

-CTAATTATAAGAGTTAACACTCGCGAGTGTTAAATAGC 5'

ClaI (#4438)

Expression of the hMKT(1-163) gene introduced into pAMG11 can be induced by a synthetic lactose-inducible promoter such as a Ps4 promoter having the following sequence:

5' GACGTCTCATAATTTTTAAAAAATTCATTTGACAAATGCTAAA-
-ATTCTTGATTAATATTCTCAATTGTGAGCGCTCACAATTTATCGAT 3'

The Ps4 promoter-induced expression of hMKT(1-163) gene is repressed by the lactose repressor (Lac I) which is a product of the *E. coli* lac I gene.

Next, an *E. coli* strain K-12 containing laq I^{q} allele was transformed with the plasmid pAMG11-hMKT(1-163). The laq I^{q} allele has a mutation within the lac I promoter which increases expression of the Lac I gene, thereby resulting in more stringent control of protein expression by the Ps4 promoter. In consequence, in the absence of lactose, expression of hMKT(1-163) is repressed by Lac I. When lactose is added, the binding of the Lac I protein to the operator site of the Ps4 promoter decreases, and the transcription of the hMKT(1-163) gene is initiated by the Ps4 promoter. The *E. coli* used as the host cell in this example has been deposited with the ATCC on November 30, 1994 under ATCC No. 69717.

The *E. coli* strain (ATCC No. 69717) was transformed with the plasmid pAMG11-hMKT(1-163) and cultured under the following culture conditions.

### (2) Culture of a recombinant E. coli strain capable of expressing hMKT(1-163) and production of TPO(1-163)/E. coli:

The obtained transformant was cultured on LB medium at 30°C for approximately 12 hours. The cells were then aseptically transferred to a fermenter containing a batch medium (20 g/L yeast extract; 3.4 g/L citric acid; 15 g/L K₂HPO₄; 15 ml Dow P2000; 5 g/L glucose; 1 g/L MgSO₄-7H₂O; 5.5 ml/L trace metals; 5.5 ml/L vitamins). The cultivation was continued until an optical density (O.D.) of the culture reached 5.0 ± 1.0 at 600 nm. Then, a first feed medium (700 g/L glucose; 6.75 g/L MgSO₄-7H₂O) was fed while adjusting a feed rate at intervals of 2 hours in accordance with an established schedule. The addition of a second feed medium (129 g/L trypticase peptone; 258 g/L yeast extract) was started when the O.D. of the culture reached 20-25 at 600 nm. The addition of the second feed medium was maintained at a constant flow rate while the addition of the first feed medium was continued to be adjusted.

The temperature was maintained at approximately 30°C during the entire cultivation. The culture was kept at about pH 7 with addition of an acid or a base if necessary. The desired dissolved oxygen level was maintained by adjusting an agitation rate, an aeration rate and an oxygen influx rate in the fermenter. When the O.D. of the culture reached 57-63 at 600 nm, the addition of a third feed medium (300 g/L lactose) was introduced into the fermenter at a constant flow rate. The addition of the first feed medium was stopped and the flow rate of the second feed medium was changed to a new constant rate. The cultivation was continued over about ten hours after initiation of the addition of the third feed medium. At the end of the cultivation, the culture was cooled to 15 ± 5°C and the cells were harvested by centrifugation. The resulting pellet was stored at a temperature of -60°C or lower.

Purification of hMKT(1-163) thus produced in *E. coli* and production of TPO(1-163)/E. coli were carried out as follows.

1800 g of the cell pellet was suspended in about 18 liters of 10 mM EDTA and passed through a high pressure homogenizer at 15,000 psi. The broken cell suspension was centrifuged and the precipitate was resuspended in 10 L of 10 mM EDTA. The suspension was centrifuged and 200 g of the precipitate was solubilized in 2 L of 10 mM Tris buffer, pH 8.7, containing 8 M guanidine hydrochloride, 10 mM DTT and 5 mM EDTA. This solution was slowly diluted in 200 L of 10 mM CAPS, pH 10.5, containing 3 M urea, 30% glycerol, 3 mM cystamine and 1 mM cysteine.

The diluted solution was stirred slowly for 16 hr at the room temperature and the pH was adjusted to 6.8. After the adjustment of pH, the solution was clarified and loaded to a 2-L CM Sepharose column equilibrated with 10 mM sodium phosphate buffer, pH 6.8, containing 1.5 M urea and 15% glycerol. After loading, the column was washed with 10 mM sodium phosphate containing 15% glycerol, pH 7.2. hMKT(1-163) was eluted with a linear gradient from 0 M to 0.5 M sodium chloride in 10 mM sodium phosphate buffer, pH 7.2.

The fractions eluted from the CM Sepharose column were concentrated using a membrane (10,000 molecular weight cut off) and simultaneously buffer-exchanged with 10 mM sodium phosphate buffer, pH 6.5. The concentrated solution (protein: about 2 mg/ml) was treated with cathepsin C (protein substrate : enzyme = 500 : 1 (molar ratio)) for 90 minutes at the ambient temperature.

The reaction mixture was then loaded to a 1.2-L SP High Performance Sepharose column equilibrated with 10 mM sodium phosphate buffer, pH 7.2, containing 15% glycerol. After loading, a TPO active protein TPO(1-163)/E. coli in which the N-terminal Met-Lys was cleaved from the hMKT(1-163) was eluted with a linear gradient from 0.1 M to 0.25 M sodium chloride in 10 mM sodium phosphate, pH 7.2.

Ammonium sulfate was added to the eluate from the SP High Performance column to a concentration of 0.6 M. The eluate was then loaded to a 1.6-L Phenyl Toyopearl column (Toso Corp., Japan) equilibrated with 10 mM sodium phosphate buffer, pH 7.2, containing 0.6 M ammonium sulfate. A peak of the TPO(1-163)/E. coli was eluted with a linear gradient from 0.6 M to 0 M ammonium sulfate in 10 mM sodium phosphate, pH 7.2.

The resulting eluate from the Phenyl Toyopearl column was concentrated using a membrane (10,000 molecular weight cut off) and simultaneously buffer-exchanged with 10 mM Tris buffer, pH 7.5, containing 5% sorbitol.

## Claims

1. A thrombopoietin (TPO)-containing lyophilized composition which comprises a TPO protein and a saccharide as a pharmaceutically acceptable additive.

2. The TPO-containing lyophilized composition according to claim 1 wherein said saccharide is contained in an amount of from 10 to 10000 parts by weight relative to one part by weight of the TPO protein contained in the TPO-containing lyophilized composition.

3. The TPO-containing lyophilized composition according to claim 1 or claim 2 wherein said saccharide is at least one saccharide selected from the group consisting of mannitol, lactose, sucrose and maltose.

4. The TPO-containing lyophilized composition according to any one of claims 1 to 3 wherein it further comprises at least one pharmaceutically acceptable additive selected from the group consisting of a surfactant, an amino acid and a protein, in addition to the TPO protein and the saccharide.

5. The TPO-containing lyophilized composition according to claim 4 wherein said surfactant is contained in an amount of from 0.01 to 10 parts by weight relative to one part by weight of the TPO protein contained in the TPO-containing lyophilized composition.

6. The TPO-containing lyophilized composition according to claim 4 wherein said amino acid is contained in an amount of from 1 to 1000 parts by weight relative to one part by weight of the TPO protein contained in the TPO-containing lyophilized composition.

7. The TPO-containing lyophilized composition according to claim 4 wherein said protein is contained in an amount of from 1 to 1000 parts by weight relative to one part by weight of the TPO contained in the TPO-containing lyophilized composition.

8. The TPO-containing lyophilized composition according to claim 4 or claim 5 wherein said surfactant is a polyoxysorbitan fatty acid ester.

9. The TPO-containing lyophilized composition according to claim 4 or claim 6 wherein said amino acid is at least one of glycine and arginine.

10. The TPO-containing lyophilized composition according to claim 4 or claim 7 wherein said protein is gelatin.


SEQUENCE LISTING

INFORMATION FOR SEQ ID NO:1:

(i) SEQUENCE CHARACTERISTICS:

(A) SEQUENCE:332 amino acids

(B) TYPE:amino acid

(ii) MOLECULE TYPE:protein

(vi) ORIGINAL SOURCE:

(A) ORGANISM:human (Homo sapiens)

(xi) SEQUENCE DESCRIPTION:SEQ ID NO:1:

Ser Pro Ala Pro Pro Ala Cys Asp Leu Arg Val Leu Ser Lys Leu Leu

1               5                   10                  15

Arg Asp Ser His Val Leu His Ser Arg Leu Ser Gln Cys Pro Glu Val

            20                  25                  30

His Pro Leu Pro Thr Pro Val Leu Leu Pro Ala Val Asp Phe Ser Leu

        35                  40                  45

Gly Glu Trp Lys Thr Gln Met Glu Glu Thr Lys Ala Gln Asp Ile Leu

    50                  55                  60

Gly Ala Val Thr Leu Leu Leu Glu Gly Val Met Ala Ala Arg Gly Gln

65                  70                  75                  80

Leu Gly Pro Thr Cys Leu Ser Ser Leu Leu Gly Gln Leu Ser Gly Gln

                85                  90                  95

Val Arg Leu Leu Leu Gly Ala Leu Gln Ser Leu Leu Gly Thr Gln Leu

            100                 105                 110

Pro Pro Gln Gly Arg Thr Thr Ala His Lys Asp Pro Asn Ala Ile Phe

        115                 120                 125

Leu Ser Phe Gln His Leu Leu Arg Gly Lys Val Arg Phe Leu Met Leu

    130                 135                 140

Val Gly Gly Ser Thr Leu Cys Val Arg Arg Ala Pro Pro Thr Thr Ala
145                 150                 155                 160
Val Pro Ser Arg Thr Ser Leu Val Leu Thr Leu Asn Glu Leu Pro Asn
                165                 170                 175
Arg Thr Ser Gly Leu Leu Glu Thr Asn Phe Thr Ala Ser Ala Arg Thr
            180                 185                 190
Thr Gly Ser Gly Leu Leu Lys Trp Gln Gln Gly Phe Arg Ala Lys Ile
        195                 200                 205
Pro Gly Leu Leu Asn Gln Thr Ser Arg Ser Leu Asp Gln Ile Pro Gly
    210                 215                 220
Tyr Leu Asn Arg Ile His Glu Leu Leu Asn Gly Thr Arg Gly Leu Phe
225                 230                 235                 240
Pro Gly Pro Ser Arg Arg Thr Leu Gly Ala Pro Asp Ile Ser Ser Gly
                245                 250                 255
Thr Ser Asp Thr Gly Ser Leu Pro Pro Asn Leu Gln Pro Gly Tyr Ser
            260                 265                 270
Pro Ser Pro Thr His Pro Pro Thr Gly Gln Tyr Thr Leu Phe Pro Leu
        275                 280                 285
Pro Pro Thr Leu Pro Thr Pro Val Val Gln Leu His Pro Leu Leu Pro
    290                 295                 300
Asp Pro Ser Ala Pro Thr Pro Thr Pro Thr Ser Pro Leu Leu Asn Thr
305                 310                 315                 320
Ser Tyr Thr His Ser Gln Asn Leu Ser Gln Glu Gly
                325                 330

INFORMATION FOR SEQ ID NO:2:
   (i) SEQUENCE CHARACTERISTICS:
      (A) SEQUENCE:1043 base pairs

(B) TYPE:nucleic acid
(C) STRANDEDNESS:double
(D) TOPOLOGY:linear
(ii) MOLECULE TYPE:synthetic DNA
(vi) ORIGINAL SOURCE:
(A) ORGANISM:human (Homo sapiens)
(xi) SEQUENCE DESCRIPTION:SEQ ID NO:2:
CTAGAAAAAA CCAAGGAGGT AATAAATA 28
ATG AAA AGT CCT GCA CCA CCT GCA TGT GAT TTA CGG GTC CTG TCT AAA 76
Met Lys Ser Pro Ala Pro Pro Ala Cys Asp Leu Arg Val Leu Ser Lys
-2 +1 5 10
CTG CTG CGC GAC TCT CAC GTG CTG CAC TCT CGT CTG TCC CAG TGC CCG 124
Leu Leu Arg Asp Ser His Val Leu His Ser Arg Leu Ser Gln Cys Pro
15 20 25 30
GAA GTT CAC CCG CTG CCG ACC CCG GTT CTG CTT CCG GCT GTC GAC TTC 172
Glu Val His Pro Leu Pro Thr Pro Val Leu Leu Pro Ala Val Asp Phe
35 40 45
TCC CTG GGT GAA TGG AAA ACC CAG ATG GAA GAG ACC AAA GCT CAG GAC 220
Ser Leu Gly Glu Trp Lys Thr Gln Met Glu Glu Thr Lys Ala Gln Asp
50 55 60
ATC CTG GGT GCA GTA ACT CTG CTT CTG GAA GGC GTT ATG GCT GCA CGT 268
Ile Leu Gly Ala Val Thr Leu Leu Leu Glu Gly Val Met Ala Ala Arg
65 70 75
GGC CAG CTT GGC CCG ACC TGC CTG TCT TCC CTG CTT GGC CAG CTG TCT 316
Gly Gln Leu Gly Pro Thr Cys Leu Ser Ser Leu Leu Gly Gln Leu Ser
80 85 90
GGC CAG GTT CGT CTG CTG CTC GGC GCT CTG CAG TCT CTG CTT GGC ACC 364
Gly Gln Val Arg Leu Leu Leu Gly Ala Leu Gln Ser Leu Leu Gly Thr

95 100 105 110

CAG CTG CCG CCA CAG GGC CGT ACC ACT GCT CAC AAG GAT CCG AAC GCT 412

Gln Leu Pro Pro Gln Gly Arg Thr Thr Ala His Lys Asp Pro Asn Ala

115 120 125

ATC TTC CTG TCT TTC CAG CAC CTG CTG CGT GGC AAA GTT CGT TTC CTG 460

Ile Phe Leu Ser Phe Gln His Leu Leu Arg Gly Lys Val Arg Phe Leu

130 135 140

ATG CTG GTT GGC GGT TCT ACC CTG TGC GTT CGT CGG GCG CCG CCA ACC 508

Met Leu Val Gly Gly Ser Thr Leu Cys Val Arg Arg Ala Pro Pro Thr

145 150 155

ACT GCT GTT CCG TCT CGT ACC TCT CTG GTT CTG ACC CTG AAC GAG CTC 556

Thr Ala Val Pro Ser Arg Thr Ser Leu Val Leu Thr Leu Asn Glu Leu

160 165 170

CCG AAC CGT ACC AGC GGC CTG CTG GAA ACC AAC TTT ACC GCG AGC GCG 604

Pro Asn Arg Thr Ser Gly Leu Leu Glu Thr Asn Phe Thr Ala Ser Ala

175 180 185 190

CGT ACC ACC GGC AGC GGC CTG CTG AAA TGG CAG CAG GGC TTT CGT GCG 652

Arg Thr Thr Gly Ser Gly Leu Leu Lys Trp Gln Gln Gly Phe Arg Ala

195 200 205

AAA ATC CCG GGC CTG CTG AAC CAG ACC AGC CGT AGC CTG GAT CAG ATC 700

Lys Ile Pro Gly Leu Leu Asn Gln Thr Ser Arg Ser Leu Asp Gln Ile

210 215 220

CCG GGC TAT CTG AAC CGT ATC CAT GAA CTG CTG AAC GGC ACC CGT GGC 748

Pro Gly Tyr Leu Asn Arg Ile His Glu Leu Leu Asn Gly Thr Arg Gly

225 230 235

CTG TTT CCG GGC CCG AGC CGT CGC ACC CTG GGC GCG CCG GAT ATC AGC 796

Leu Phe Pro Gly Pro Ser Arg Arg Thr Leu Gly Ala Pro Asp Ile Ser

240 245 250

TCT GGC ACC AGC GAT ACC GGC AGC CTG CCG CCG AAC CTG CAG CCG GGC 844
Ser Gly Thr Ser Asp Thr Gly Ser Leu Pro Pro Asn Leu Gln Pro Gly
255 260 265 270
TAT AGC CCG AGC CCG ACC CAT CCG CCG ACC GGC CAG TAT ACC CTG TTT 892
Tyr Ser Pro Ser Pro Thr His Pro Pro Thr Gly Gln Tyr Thr Leu Phe
275 280 285
CCG CTG CCG CCG ACC CTG CCG ACC CCG GTG GTT CAG CTG CAT CCG CTG 940
Pro Leu Pro Pro Thr Leu Pro Thr Pro Val Val Gln Leu His Pro Leu
290 295 300
CTG CCG GAT CCG AGC GCG CCG ACC CCG ACC CCG ACC AGC CCG CTG CTG 988
Leu Pro Asp Pro Ser Ala Pro Thr Pro Thr Pro Thr Ser Pro Leu Leu
305 310 315
AAC ACC AGC TAT ACC CAT AGC CAG AAC CTG AGC CAG GAA GGC 1030
Asn Thr Ser Tyr Thr His Ser Gln Asn Leu Ser Gln Glu Gly
320 325 330
TAATGAAGCT TGA 1043

INFORMATION FOR SEQ ID NO:3:
(i) SEQUENCE CHARACTERISTICS:
(A) SEQUENCE:1721 base pairs
(B) TYPE:nucleic acid
(C) STRANDEDNESS:double
(D) TOPOLOGY:linear
(ii) MOLECULE TYPE:cDNA to mRNA
(vi) ORIGINAL SOURCE:
(A) ORGANISM:human (Homo sapiens)
(B) TISSUE TYPE:liver
(xi) SEQUENCE DESCRIPTION:SEQ ID NO:3:

GCGGCACGAG GGGGGTGTCT GGCTGGCGTG GCTCCCTGTT TGGGGCCTCT CCCCTGAATC 60

CTTCCTGGGG CCATGGAGGC CAGACAGACA CCCCGGCCAG A 101

ATG GAG CTG ACT GAA TTG CTC CTC GTG GTC ATG CTT CTC CTA ACT GCA 149



-20 -15 -10

AGG CTA ACG CTG TCC AGC CCG GCT CCT CCT GCT TGT GAC CTC CGA GTC 193



-5 1 5 10

CTC AGT AAA CTG CTT CGT GAC TCC CAT GTC CTT CAC AGC AGA CTG AGC 245



15 20 25

CAG TGC CCA GAG GTT CAC CCT TTG CCT ACA CCT GTC CTG CTG CCT GCT 293



30 35 40

GTG GAC TTT AGC TTG GGA GAA TGG AAA ACC CAG ATG GAG GAG ACC AAG 341



45 50 55

GCA CAG GAC ATT CTG GGA GCA GTG ACC CTT CTG CTG GAG GGA GTG ATG 389



60 65 70 75

GCA GCA CGG GGA CAA CTG GGA CCC ACT TGC CTC TCA TCC CTC CTG GGG 437



80 85 90

CAG CTT TCT GGA CAG GTC CGT CTC CTC CTT GGG GCC CTG CAG AGC CTC 485



95 100 105

CTT GGA ACC CAG CTT CCT CCA CAG GGC AGG ACC ACA GCT CAC AAG GAT 533



110 115 120
CCC AAT GCC ATC TTC CTG AGC TTC CAA CAC CTG CTC CGA GGA AAG GTG 581

125 130 135
CGT TTC CTG ATG CTT GTA GGA GGG TCC ACC CTC TGC GTC AGG CGG GCC 629

140 145 150 155
CCA CCC ACC ACA GCT GTC CCC AGC AGA ACC TCT CTA GTC CTC ACA CTG 677

160 165 170
AAC GAG CTC CCA AAC AGG ACT TCT GGA TTG TTG GAG ACA AAC TTC ACT 725

175 180 185
GCC TCA GCC AGA ACA ACT GGC TCT GGG CTT CTG AAG TGG CAG CAG GGA 773

190 195 200
TTC AGA GCC AAG ATT CCT GGT CTG CTG AAC CAA ACC TCC AGG TCC CTG 821

205 210 215
GAC CAA ATC CCC GGA TAC CTG AAC AGG ATA CAC GAA CTC TTG AAT GGA 869

220 225 230 235
ACT CGT GGA CTC TTT CCT GGA CCC TCA CGC AGG ACC CTA GGA GCC CCG 917

240 245 250
GAC ATT TCC TCA GGA ACA TCA GAC ACA GGC TCC CTG CCA CCC AAC CTC 965

255 260 265

CAG CCT GGA TAT TCT CCT TCC CCA ACC CAT CCT CCT ACT GGA CAG TAT     1013



        270                 275                 280

ACG CTC TTC CCT CTT CCA CCC ACC TTG CCC ACC CCT GTG GTC CAG CTC     1061



    285                 290                 295

CAC CCC CTG CTT CCT GAC CCT TCT GCT CCA ACG CCC ACC CCT ACC AGC     1109



300                 305                 310                 315

CCT CTT CTA AAC ACA TCC TAC ACC CAC TCC CAG AAT CTG TCT CAG GAA     1157



                320                 325                 330

GGG TAAGGTTCTC AGACACTGCC GACATCAGCA TTGTCTCGTG TACAGCTCCC          1210



TTCCCTGCAG GGCGCCCCTG GGAGACAACT GGACAAGATT TCCTACTTTC TCCTGAAACC   1270

CAAAGCCCTG GTAAAAGGGA TACACAGGAC TGAAAAGGGA ATCATTTTTC ACTGTACATT   1330

ATAAACCTTC AGAAGCTATT TTTTTAAGCT ATCAGCAATA CTCATCAGAG CAGCTAGCTC   1390

TTTGGTCTAT TTTCTGCAGA AATTTGCAAC TCACTGATTC TCTACATGCT CTTTTTCTGT   1450

GATAACTCTG CAAAGGCCTG GGCTGGCCTG GCAGTTGAAC AGAGGGAGAG ACTAACCTTG   1510

AGTCAGAAAA CAGAGAAAGG GTAATTTCCT TTGCTTCAAA TTCAAGGCCT TCCAACGCCC   1570

CCATCCCCTT TACTATCATT CTCAGTGGGA CTCTGATCCC ATATTCTTAA CAGATCTTTA   1630

CTCTTGAGAA ATGAATAAGC TTTCTCTCAG AAATGCTGTC CCTATACACT AGACAAAACT   1690

GAAAAAAAAA AAAAAAAAAA AAAAAAAAAA A                                  1721

INFORMATION FOR SEQ ID NO:4:

(i) SEQUENCE CHARACTERISTICS:

(A) SEQUENCE:1086 base pairs

(B) TYPE:nucleic acid

(C) STRANDEDNESS:double

(D) TOPOLOGY:linear

(ii) MOLECULE TYPE:cDNA to mRNA

(vi) ORIGINAL SOURCE:

(A) ORGANISM:human (Homo sapiens)

(B) TISSUE TYPE:liver

(xi) SEQUENCE DESCRIPTION:SEQ ID NO:4:

GGCCAGCCAG ACACCCCGGC CAGA 24

ATG GAG CTG ACT GAA TTG CTC CTC GTG GTC ATG CTT CTC CTA ACT GCA 72



-20 -15 -10

AGG CTA ACG CTG TCC AGC CCG GCT CCT CCT GCT TGT GAC CTC CGA GTC 120



-5 1 5 10

CTC AGT AAA CTG CTT CGT GAC TCC CAT GTC CTT CAC AGC AGA CTG AGC 168



15 20 25

CAG TGC CCA GAG GTT CAC CCT TTG CCT ACA CCT GTC CTG CTG CCT GCT 216



30 35 40

GTG GAC TTT AGC TTG GGA GAA TGG AAA ACC CAG ATG GAG GAG ACC AAG 264



45 50 55

GCA CAG GAC ATT CTG GGA GCA GTG ACC CTT CTG CTG GAG GGA GTG ATG 312



60 65 70 75

GCA GCA CGG GGA CAA CTG GGA CCC ACT TGC CTC TCA TCC CTC CTG GGG 360



80 85 90
CAG CTT TCT GGA CAG GTC CGT CTC CTC CTT GGG GCC CTG CAG AGC CTC 408

95 100 105
CTT GGA ACC CAG CTT CCT CCA CAG GGC AGG ACC ACA GCT CAC AAG GAT 456

110 115 120
CCC AAT GCC ATC TTC CTG AGC TTC CAA CAC CTG CTC CGA GGA AAG GTG 504

125 130 135
CGT TTC CTG ATG CTT GTA GGA GGG TCC ACC CTC TGC GTC AGG CGG GCC 552

140 145 150 155
CCA CCC ACC ACA GCT GTC CCC AGC AGA ACC TCT CTA GTC CTC ACA CTG 600

160 165 170
AAC GAG CTC CCA AAC AGG ACT TCT GGA TTG TTG GAG ACA AAC TTC ACT 648

175 180 185
GCC TCA GCC AGA ACT ACT GGC TCT GGG CTT CTG AAG TGG CAG CAG GGA 696

190 195 200
TTC AGA GCC AAG ATT CCT GGT CTG CTG AAC CAA ACC TCC AGG TCC CTG 744

205 210 215
GAC CAA ATC CCC GGA TAC CTG AAC AGG ATA CAC GAA CTC TTG AAT GGA 792

220 225 230 235

ACT CGT GGA CTC TTT CCT GGA CCC TCA CGC AGG ACC CTA GGA GCC CCG 840



240 245 250

GAC ATT TCC TCA GGA ACA TCA GAC ACA GGC TCC CTG CCA CCC AAC CTC 888



255 260 265

CAG CCT GGA TAT TCT CCT TCC CCA ACC CAT CCT CCT ACT GGA CAG TAT 936



270 275 280

ACG CTC TTC CCT CTT CCA CCC ACC TTG CCC ACC CCT GTG GTC CAG CTC 984



285 290 295

CAC CCC CTG CTT CCT GAC CCT TCT GCT CCA ACG CCC ACC CCT ACC AGC 1032



300 305 310 315

CCT CTT CTA AAC ACA TCC TAC ACC CAC TCC CAG AAT CTG TCT CAG GAA 1080



320 325 330

GGG TAA 1086



INFORMATION FOR SEQ ID NO:5:

(i) SEQUENCE CHARACTERISTICS:

(A) SEQUENCE:535 base pairs

(B) TYPE:nucleic acid

(C) STRANDEDNESS:double

(D) TOPOLOGY:linear

(ii) MOLECULE TYPE:synthetic DNA

(vi) ORIGINAL SOURCE:
(A) ORGANISM:human (Homo sapiens)
(xi) SEQUENCE DESCRIPTION:SEQ ID NO:5:
CTAGAAAAAA CCAAGGAGGT AATAAATA 28
ATG AAA AGT CCT GCA CCA CCT GCA TGT GAT TTA CGG GTC CTG TCT AAA 76
Met Lys Ser Pro Ala Pro Pro Ala Cys Asp Leu Arg Val Leu Ser Lys
+1 5 10
CTG CTG CGC GAC TCT CAC GTG CTG CAC TCT CGT CTG TCC CAG TGC CCG 124
Leu Leu Arg Asp Ser His Val Leu His Ser Arg Leu Ser Gln Cys Pro
15 20 25 30
GAA GTT CAC CCG CTG CCG ACC CCG GTT CTG CTT CCG GCT GTC GAC TTC 172
Glu Val His Pro Leu Pro Thr Pro Val Leu Leu Pro Ala Val Asp Phe
35 40 45
TCC CTG GGT GAA TGG AAA ACC CAG ATG GAA GAG ACC AAA GCT CAG GAC 220
Ser Leu Gly Glu Trp Lys Thr Gln Met Glu Glu Thr Lys Ala Gln Asp
50 55 60
ATC CTG GGT GCA GTA ACT CTG CTT CTG GAA GGC GTT ATG GCT GCA CGT 268
Ile Leu Gly Ala Val Thr Leu Leu Leu Glu Gly Val Met Ala Ala Arg
65 70 75
GGC CAG CTT GGC CCG ACC TGC CTG TCT TCC CTG CTT GGC CAG CTG TCT 316
Gly Gln Leu Gly Pro Thr Cys Leu Ser Ser Leu Leu Gly Gln Leu Ser
80 85 90
GGC CAG GTT CGT CTG CTG CTC GGC GCT CTG CAG TCT CTG CTT GGC ACC 364
Gly Gln Val Arg Leu Leu Leu Gly Ala Leu Gln Ser Leu Leu Gly Thr
95 100 105 110
CAG CTG CCG CCA CAG GGC CGT ACC ACT GCT CAC AAG GAT CCG AAC GCT 412
Gln Leu Pro Pro Gln Gly Arg Thr Thr Ala His Lys Asp Pro Asn Ala
115 120 125

ATC TTC CTG TCT TTC CAG CAC CTG CTG CGT GGC AAA GTT CGT TTC CTG 460

Ile Phe Leu Ser Phe Gln His Leu Leu Arg Gly Lys Val Arg Phe Leu

130 135 140

ATG CTG GTT GGC GGT TCT ACC CTG TGC GTT CGT CGG GCG CCG CCA ACC 508

Met Leu Val Gly Gly Ser Thr Leu Cys Val Arg Arg Ala Pro Pro Thr

145 150 155

ACT GCT GTT CCG TCT TAATGAAAGC TT 535

Thr Ala Val Pro Ser

160